Europäisches Patentamt

European Patent Office

Office européen des brevets

(11) Publication number: **0 137 743**
A2

# (12) EUROPEAN PATENT APPLICATION

(21) Application number: **84306236.5**

(22) Date of filing: **12.09.84**

(51) Int. Cl.⁴: **A 61 K 47/00**
A 61 L 15/03, A 61 K 31/195
A 61 K 33/14

(30) Priority: **13.09.83 GB 8324487**
**13.09.83 GB 8324488**

(43) Date of publication of application:
**17.04.85 Bulletin 85/16**

(84) Designated Contracting States:
**BE CH DE FR GB IT LI NL SE**

(71) Applicant: **Ed. Geistlich Söhne A.G. für Chemische Industrie**

**CH-6110 Wolhusen Lucerne(CH)**

(72) Inventor: **Geistlich, Peter**
**Seldwyla**
**Stansstadt(CH)**

(74) Representative: **Holmes, Michael John et al,**
**Frank B. Dehn & Co. Imperial House 15-19 Kingsway**
**London WC2B 6UZ(GB)**

(54) Topically administrable pharmaceutical compositions.

(57) The invention provides pharmaceutical compositions in solid or semi-solid form for topical application to external wounds to enhance tissue regeneration comprising calcium and potassium ions, physiologically acceptable counter ions therefor and one or more topical pharmaceutical carriers or excipients. The compositions include in particular, semi solid gels containing amino acids and solid gels adapted to release wound-healing substances.

0137743

DF.142-890CD

TOPICALLY ADMINISTRABLE PHARMACEUTICAL COMPOSITIONS

This invention relates to pharmaceutical compositions in forms suitable for topical administration to the human or animal body to promote the healing of wounds.

It is necessary with patients with extensive tissue damage, particularly wounds (including wounds resulting from injuries or surgical operations and burns), to protect the wound while tissue regrowth occurs. For this purpose various topically administered preparations, such as gels and ointments, have been used which maintain an acceptable environment at the tissue regeneration site, for example in terms of temperature and moisture. Such preparations may contain antibacterial agents to reduce or avoid infection of the wound site or growth promotors to enhance tissue regeneration.

It is an object of the present invention to provide a solid or semi-solid topically administrable composition capable of enhancing tissue regeneration at external wound sites.

It has been indicated in European Patent Application Publication No 47647A that wound healing may be promoted by the topical application of a semi-solid aqueous gel containing the essential and semi-essential amino acids which serve as nutrients for the regenerating tissue. Although inorganic ions including potassium are mentioned in such compositions there is no reference to calcium ions.

We have found that tissue regeneration is enhanced by the inclusion of both calcium and potassium ions in pharmaceutical preparations administered topically to external wound sites.

According to one aspect of the invention we therefore provide pharmaceutical compositions in solid or semi-solid form for topical application
to external wounds to enhance tissue regeneration
comprising calcium and potassium ions, physiologically
acceptable, compatible counter-ions therefor and
one or more topical pharmaceutical carriers or excipients.

The solid and semi-solid pharmaceutical compositions
of the invention are advantageously in the form of
smearable gels, creams, emulsions, balms or salves,
powders, films or webs. The term "solid" in this
context relates to gels having some resilience or
dimensional stability, in contrast to "semi-solid"
gels which are smearable and do not have such dimensional
stability. The solid compositions particularly advantageously
comprise as the carrier a solid (as distinct from
semi-solid) water-permeable aqueous polymer gel film
or web such as the acrylamide - agar agar films described
in UK Patent Specification GB 2036042A of Max-Planck-
Gesellschaft zur Forderung der Wissenschaften E.V.
It is, naturally desirable that such films or webs,
which essentially act as bandages which are capable
of releasing wound-healing medicaments, should be
structurally stable, that is they should not be dissolved
or resolved by the fluids exuded by the wound. Furthermore,
it is often desirable to apply such gel materials
in powder form to provide a layer of aqueous gel
over the wound and in this case also, it is preferred
that the gel is not soluble or resorbable.

The compositions of the invention suitably
contain calcium ions in concentrations of up to about
50 mMol, preferably 1 to 40 mMol and most preferably
from 25 to 35 mMol. The compositions suitably contain
potassium ions in concentration of up to about 50
mMol, advantageously 0.1 to 40 mMol, preferably 5
to 40 mMol and especially preferably 25 to 35 mMol.
However, the concentration of potassium ions should
not be at toxic levels and thus should not generally
exceed about 50 mMol.

The compositions of the invention, particularly when in the form of aqueous gels or water-permeable aqueous polymer films or webs are preferably at substantially neutral pH to enhance their compatabiity with the wound site and will often be isotonic. The compositions may thus contain further ionic material. Suitable cations include magnesium, zinc and sodium; nevertheless it is preferred that sodium ion concentrations should be low and sodium ion free or substantially sodium ion free compositions are particularly preferred.

The counter-ions both for the potassium and calcium ions and for any further cations included (e.g. to adjust the ionic strength of the compositions) should clearly be physiologically acceptable anions which are compatible in the sense that they do not form insoluble compounds with calcium or potassium ions (or any other cations present); particularly suitable counter-ions include acetate and chloride. In the case of smearable gels containing amino acids, suitable counter ions include anions from acidic amino acids such as glutamic or aspartic acid.

To adjust the pH of the composition to the desired level, physiologically acceptable, compatible acids or bases should be used, i.e. compounds which do not form insoluble salts or complexes with any of the other components. Particularly suitable acids for use in this respect are acetic, malic, hydrochloric and hydrobromic acids.

The compositions of the invention may optionally further contain growth promoting agents, such as for example fibronectin, a growth factor in the production of the protein "net" over a wound in the first stages of healing, or calmodulin a calcium regulator, or chelating agents, such as for example EGTA (ethylene glycol tetraacetic acid).

The compositions of the invention suitably include as pharmaceutical carriers or excipients compounds or materials which enable the compositions to be presented in topically administrable forms such as those recited above. Thus those carriers and excipients may be selected from those known in the art for the formulation of preparations suitable for treatment of external wounds.

In general, the compositions of the invention may be produced by the incorporation of calcium and potassium ions into known OTC emulsions, ointments, creams and gels such as baby creams, mild antiseptic ointments, haemorrhoid creams etc., into water-permeable aqueous polymer gels or into cellulose ester, e.g. carboxymethylcellulose (CMC), microcrystalline cellulose, polysaccharide (e.g. alginate, agarose, tragacanth etc), or polyvinylpyrrolidone based gels.

The solid water-permeable aqueous polymer gel compositions may advantageously comprise at least one gellable polysaccharide and/or protein or polypeptide and a cross-linked polymer of a hydrophilic acrylic acid or methacrylic acid derivative. The said hydrophilic-derivative is preferably an amide such as acrylamide. The gellable substance is preferably agar agar. The gel composition may be formed by polymerising the hydrophilic acrylic or methacrylic acid derivative together with a bifunctional acrylic cross-linking agent in admixture with a colloidal dispersion of the gellable substance.

The semi-solid pharmaceutical compositions of the invention are preferably in smearable gel-like form. By "semi-solid aqueous gel form" is meant a gel-like form which is spreadable or smearable, having the consistency of an ointment or cream. Such compositions may be in the form of colloidal dispersions of materials such as carboxymethylcellulose, agarose etc which have little or none of the resilience or elasticity of a true gel but are particularly beneficial in releasing nutrients into the wound, as compared with solid or rigidly gelatinous nutrient formulations.

In general, the total concentration of amino acids in the compositions of the invention should
be in the range 3-17% by weight. However, the overall
quantity of amino acid material partly depends on
the nature of the individual amino acids. In many
intravenous nutrient solutions, the total nitrogen
supplied is predominantly in the form of simple amino
acids such as glycine, alanine and glutamic acid.
The body is capable of using these amino acids to
synthesise a number of the non-essential amino acids
present in human tissue. In general, however, this
approach is less efficient than using a mixture of
amino acids which approximates relatively closely
to the proportions of the amino acids found in human
tissues. It seems likely that the mechanisms available
to the body for synthesis of other amino acids from
glycine, alanine and glutamic acid may not be effective
at the site of granulation of new tissue. For that
reason, the amino acids are, in one embodiment of
the invention, preferably substantially in the proportions
found in human tissue and in that it is not intended
that the quantities of glycine, alanine and glutamic
acid should be high enough to provide significant
levels of other amino acids, the overall concentration
of amino acids in the gel according to the invention
is preferably in the range 3 to 6% by weight.

It should be noted, however, that although
the primary task of wound healing may be to regenerate
skin, which consists largely of fibrous tissue, crude
amino acid preparations derived by hydrolysis of
collagen or elastin, which may be relatively inexpensive,
do not contain all the essential amino acids and
may contain peptide materials giving rise to reactions
at the site of application.

When the composition of the invention is in
the form of a hypertonic gel, due to relatively high
concentrations of amino acids, the wound healing
process may be promoted due to increased absorption
of liquid secretion into the gel and increased movement of amino acids out of the gel.  On the other hand,
where the gel is substantially isotonic, it is more
compatible with the granulating tissue.

The amino acids used in the compositions of
the invention are preferably present in the following
quantities in g/kg composition.

| | | |
|---|---|---|
| L-Isoleucine | 1.3 – 5.7 | |
| L-Leucine | 2.1 – 8.8 | |
| L-Lysine HCl | 3.1 – 8.5 | |
| L-Methionine | 1.8 – 7.5 | Essential |
| L-Phenylalanine | 3.2 – 7.5 | amino acids |
| L-Threonine | 1.0 – 4.0 | |
| L-Tryptophan | 0.5 – 1.7 | |
| L-Valine | 1.6 – 6.7 | |
| | | |
| L-Arginine | 2.1 – 9.7 | Semi-essential |
| L-Histidine | 1.1 – 5.8 | amino acids |
| L-Alanine | 2.9 – 15.6 | |
| L-Glutamic acid | 1.9 – 21.6 | |
| Glycine | 2.0 – 25.0 | |
| L-Proline | 2.0 – 15.0 | |
| L-Ornithine – L-Aspartate | 1.0 – 8.5 | |
| L-Serine | 1.6 – 7.3 | |

It is highly preferred that all the essential
and semi-essential amino acids are present in the
compositions of the invention.

A preferred range of amino acid concentrations
in g/kg composition is as follows:-

| | |
|---|---|
| L-Isoleucine | 1.8 – 5.4 |
| L-Leucine | 2.7 – 8.3 |
| L-Lysine HCl | 3.2 – 9.6 |
| L-Methionine | 2.2 – 6.8 |
| L-Phenylalanine | 3.2 – 9.8 |
| L-Threonine | 1.5 – 4.5 |
| L-Tryptophan | 0.6 – 2.0 |

| L-Valine | 2.1 - 6.3 |
| L-Arginine | 4.3 - 12.9 |
| L-Histidine | 1.0 - 3.2 |
| L-Alanine | 4.7 - 14.1 |
| L-Glutamic acid | 0.9 - 2.9 |
| Glycine | 2.0 - 6.2 |
| L-Proline | 5.6 - 16.9 |
| L-Ornithine - L-Aspartate | 0.9 - 2.9 |
| L-Serine | 1.0 - 3.5 |

It is particularly preferred that the compositions of the invention contain malic acid. This is directly beneficial in the regenerative process. The concentration of malic acid in the composition is preferably in the range 0.2 - 0.6% by weight. The pH of the composition is preferably approximately neutral, i.e. in the range 6.5 - 8, preferably 7.0 - 7.5. It will be appreciated that in this pH range, many of the amino acids and the malic acid will be present partially in the form of salts.

The compositions of the invention suitably include as pharmaceutical carriers or excipients compounds or materials which enable the compositions to be presented in topically administrable semi-solid aqueous gel forms. Carboxymethylcellulose is particularly suitably used as a gel-forming agent. However, other cellulose derivatives such as microcrystalline cellulose as well as polysaccharides such as alginate and agarose, tragacanth are also suitable as gel-forming agents.

It may be preferable for the gel-forming agent to be resorbable, particularly where there is a possibility of occlusion of the gel as the wound heals; resorbable gel-forming agents include dextrin, pectin and polyvinyl pyrrolidone. The gel may, if required, be made thicker and/or stiffer by addition of a relatively resilient gel-forming material such as a cross-linked fibrous protein, e.g. gelatin or collagen cross-linked with formaldehyde. Thus gelatin may be swelled with water to make an aqueous gel, contacted with formalin to effect cross-linking and subjected to washing to remove excess formaldehyde; the cross-linked gelatin may then be added to an amino acid solution, conveniently after removal of some or all of the water from the gel.

Where carboxymethyl cellulose is used as a gel-forming agent, this will normally be present as a salt. The sodium salt is readily available but where it is desired to reduce the concentration of sodium ions in the composition, another watersoluble salt may be desirable, for example the potassium salt or a salt with a physiologically acceptable organic base such as ethanolamine or triethanolamine or one of the basic amino acids, such as arginine or lysine, included in the amino acid component of the composition.

According to a further aspect of the invention we provide a method of treatment of the human or animal body to enhance tissue regeneration at an external wound site, the method comprising the topical administration at the external wound site of a solid or semi-solid pharmaceutical composition comprising calcium and potassium ions together with physiologically acceptable, compatible counter-ions therefor and at least one topical pharmaceutical carrier or excipient.

According to a yet further aspect of the invention we provide the use of solid or semi-solid topically administrable pharmaceutical compositions comprising calcium and potassium ions together with physiologically acceptable, compatible counter-ions therefor and at least one topical pharmaceutical carrier or excipient for the treatment of humans or animals to enhance tissue regeneration at external wound sites.

The compositions of the invention are intended for topical application to external wound sites, e.g. burns, accidental or surgical wounds and lesions, preferably at relatively long intervals, e.g. every 12-24 hours. When a fresh application is made, if the wound site is to be cleaned before the fresh application this should prefearbly be effected without exposing the regenerating tissue to temperatures significantly above or below body temperature and without abrasion of the site. Where the wound site is large and there is a significant danger of infection, it may be desirable to disinfect the site before application of the compositions of the invention. Suitable antibacterial substances for this purpose include Noxythioline, Taurolidine, iodine-providone, chlorhexidine and antibiotics suitable for local administration such as amfomycin, bacitracin, neomycin, tyrothricin, polymyxin, as well as tetracyclines and amino glycosides.

The invention will now be further illustrated by the following Examples:

## Examples 1-6

3.5g of acrylamide and 91mg of methylenebisacrylamide are dissolved in 50 ml of distilled water. 2g of agarose or agar-agar are dissolved in 50 ml of distilled water on the water bath at 100°C, allowed to cool to 60°C, and, after the addition of 60 μl of N,N,N',N'-tetramethylenediamine (TEMED) and 45mg of peroxydisulfate, mixed with the acrylamide solution. The solution is poured immediately on to a glass plate measuring 12.5 x 26 cm and having a rim 2mm high which has been preheated on a hot plate to 65°C. The latter is sealed with a glass cover such that no air bubbles are included. The cell is held for about 30 minutes at 56°C, until it is ascertained that the acrylamide has polymerized. After cooling, the plate is allowed to cure for at least 24 hours at 4°C. After removal, the gel is washed several times with one of the six solutions set out in the following Table to remove unpolymerised material and to impregnate the gel with the aqueous composition under test. The gel sheets were subsequently granulated.

## TABLE

| Example | Potassium ion Concentration (mMol) | Calcium ion Concentration (mMol) |
|---|---|---|
| 1 | 4 | 0.3 |
| 2 | 40 | 0.3 |
| 3 | 4 | 3 |
| 4 | 40 | 3 |
| 5 | 4 | 30 |
| 6 | 40 | 30 |

The compositions of Examples 1-6 were evaluated in tests on guinea pigs. In these tests a wound was created on the animals' backs and the granulated gel compositions were applied topically to the wound sites. On subsequent removal of the composition, the granulation tissue that had formed on the wound site was removed and weighed.

It was found that a marked increase in granulation was observed on application of the above calcium/potassium compostions as compared to the controls.

EXAMPLE 7

1.0 kg of gel contains:

|  | g | g |
|---|---|---|
| Sodium-carboxy-methylcellulose | 13.000 | |
| Glycerol BP | 10.000 | |
| Propyleneglycol | 10.000 | |
| | 33.000 | 33.000 |
| | | |
| L-Isoleucine | 3.586 | |
| L-Leucine | 5.444 | |
| L-Lysine HCl | 6.382* | |
| L-Methionine | 4.506 | |
| L-Phenylalanine | 6.459 | |
| L-Threonine | 2.998 | |
| L-Tryptophan | 1.315 | |
| L-Valine | 4.206 | |
| L-Arginine | 8.635 | |
| L-Histidine | 2.060 | |
| L-Alanine | 9.380 | |
| L-Glutamic Acid | 1.876 | |
| Glycine | 4.129 | |
| L-Proline | 11.265 | |
| L-Ornithine - L-Aspartate | 1.876 | |
| L-Serine | 2.253 | |
| | 76.370 | 109.370 |

|  | mmol** | | |
|---|---|---|---|
| Calcium chloride dihydrate | 30.0 | 4.411 | |
| Magnesium acetate anhydrate | 2.34 | 0.333 | |
| Potassium chloride | 40.0 | 2.982 | |
| | | 7.726 | 117.096 |
| Malic acid | | 4.318 | 121.414 |
| Aqua dest. | | ad | 1000.000 |

* (Base 5.016)

** (Chloride ions  Cl⁻: 100.0  mmol
   Acetate ions  Ac⁻:  4.68 mmol)

The glycerol and propylene glycol are added
to the carboxymethylcellulose and mixed to produce
a smooth dispersion.  The amino acids, trace minerals

0137743

and malic acid are dissolved in most of the water and the pH is adjusted to 7.2 with hydroxide. The above dispersion is added to the amino acid solution with continuous stirring, until free from undispersed solids and entrained air. The remaining water is then added to make up to the final volume. The resulting transparent, semi-solid gel is then autoclaved at 115°C - 118°C for at least 30 minutes.

J

0137743

EXAMPLE 8

1.0 kg of gel contains:

|  | g | g |
|---|---|---|
| Potassium-carboxy-methylcellulose | 13.000 | |
| Glycerol BP | 10.000 | |
| Propyleneglycol | 10.000 | |
| | 33.000 | 33.000 |
| | | |
| L-Isoleucine | 3.586 | |
| L-Leucine | 5.444 | |
| L-Lysine HCl | 6.382* | |
| L-Methionine | 4.506 | |
| L-Phenylalanine | 6.459 | |
| L-Threonine | 2.998 | |
| L-Tryptophan | 1.315 | |
| L-Valine | 4.206 | |
| L-Arginine | 8.635 | |
| L-Histidine | 2.060 | |
| L-Alanine | 9.380 | |
| L-Glutamic Acid | 1.876 | |
| Glycine | 4.129 | |
| L-Proline | 11.265 | |
| L-Ornithine - L-Aspartate | 1.876 | |
| L-Serine | 2.253 | |
| | 76.370 | 109.370 |

|  | mmol** | g | g |
|---|---|---|---|
| Calcium chloride dihydrate | 30.0 | 4.411 | |
| Magnesium acetate anhydrate | 2.34 | 0.333 | |
| Potassium chloride | 29.0 | 2.162 | |
| | | 6.906 | 116.276 |
| Malic acid | | 4.318 | 120.594 |
| Aqua dest. | | ad | 1000.000 |

\* (Base 5.016)

\** (Chloride ions Cl⁻: 89.0 mmol
Acetate ions Ac⁻: 4.68 mmol)

The gel is made up as in Example 7.

0137743

EXAMPLE 9

1.0 kg of gel contains:

| | g | g |
|---|---|---|
| Sodium-carboxy-methylcellulose | 13.000 | |
| Glycerol BP | 10.000 | |
| Propyleneglycol | 10.000 | |
| | 33.000 | 33.000 |
| | | |
| L-Isoleucine | 3.586 | |
| L-Leucine | 5.444 | |
| L-Lysine HCl | 6.382* | |
| L-Methionine | 4.506 | |
| L-Phenylalanine | 6.459 | |
| L-Threonine | 2.998 | |
| L-Tryptophan | 1.315 | |
| L-Valine | 4.206 | |
| L-Arginine | 8.635 | |
| L-Histidine | 2.060 | |
| L-Alanine | 9.380 | |
| L-Glutamic Acid | 1.876 | |
| Glycine | 4.129 | |
| L-Proline | 11.265 | |
| L-Ornithine - L-Aspartate | 1.876 | |
| L-Serine | 2.253 | |
| | 76.370 | 109.370 |
| | | |
| Calcium chloride hexahydrate | 4.000 | |
| Sodium chloride | 6.000 | |
| Potassium chloride | 4.000 | |
| | 14.000 | 123.370 |
| | | |
| Malic acid | 4.318 | 127.688 |
| | | |
| Aqua dest. | ad | 1000.000 |

* (Base 5.016)

The gel is made up as in Example 7.

0137743

Example 10

Edible gelatin (125g) was dispersed in 1% aqueous taurolidine (1250ml) for about 10 minutes and subsequently warmed to 60°C with stirring. Aqueous formaldehyde (36%; 12ml) was added to the liquid gel with stirring. The mixture was further stirred at 60°C for 10-15 minutes and then poured into clean pre-heated polyvinyl-chloride tubes (diameter 14mm). The tubes were cooled overnight and cut into 15 cm lengths and cut open. The transparent rods so obtained were then washed in a 1% taurolidine solution for about 4 hours in order to remove excess formaldehyde. The formaldehyde was quantitatively detected by gas chromatography (GC-WLD) and the washing was continued until no further free formaldehyde diffused into the wash water. The detection limit for free formaldehyde by this method was 0.003%.

The rods were granulated in a Zyliss electric mincer, with sieve openings of 4.5mm and dried in vacuo sufficiently to be milled to a fine powder.

5mg of milled dried gel prepared as above were added to 100g of the aqueous formulation of Example 1 and stirred until a smooth gel formed.

CLAIMS:

1.    Pharmaceutical compositions in solid or semi-solid form for topical application to external wounds to enhance tissue regeneration comprising calcium and potassium ions, physiologically acceptable counter ions therefor and one or more topical pharmaceutical carriers or excipients.

2.    Compositions as claimed in claim 1 in the form of solid aqueous gels.

3.    Compositions as claimed in claim 2 in which said gel comprises at least one gellable polysaccharide and/or protein or polypeptide and a cross-linked polymer of a hydrophilic acrylic acid or methacrylic acid derivative.

4.    Compositions as claimed in claim 2 in which said gel comprises agar agar and cross-linked polyacrylamide.

5.    Compositions as claimed in claim 1 in the semi-solid aqueous gel form comprising some or all of the essential and semi-essential amino acids.

6.    Compositions as claimed in claim 5 containing malic acid and in which the total concentration of amino acids therein is 3-17% by weight.

7.    Compositions as claimed in any of the preceding claims containing calcium ions and potassium ions each in concentrations of up to 50mM.

8.    Compositions as claimed in claim 7 in containing calcium and potassium ions each in concentrations in the range 5 to 40mMol.

9.    Compositions as claimed in any of the preceding claims substantially free from sodium ions.

10.    The use of compositions as claimed in claim 1 for the treatment of humans and animals to enhance tissue regeneration at external wound sites.